# EUROPEAN PATENT SPECIFICATION

(11) **EP 3 468 496 B1**
(45) Date of publication and mention of the grant of the patent: **28.10.2020**
(21) Application number: 17727598.9
(22) Date of filing: 05.06.2017
(51) Int. Cl.: A61B 18/18

(54) **ELECTROSURGICAL DEVICE WITH INTEGRATED MICROWAVE SOURCE**
ELEKTROCHIRURGISCHE VORRICHTUNG MIT INTEGRIERTER MIKROWELLENQUELLE
DISPOSITIF ÉLECTRO-CHIRURGICAL À SOURCE DE MICRO-ONDES INTÉGRÉE

(30) Priority: 13.06.2016 GB 201610252
(43) Date of publication of application: 17.04.2019
(73) Proprietor: Creo Medical Limited, Wales NP16 5UH (GB)
(72) Inventor: HANCOCK, Christopher Paul, Bath Bath and North East Somerset BA1 4LN (GB); BURN, Patrick, Chepstow Monmouthshire NP16 5UH (GB)
(74) Representative: Mewburn Ellis LLP
(86) International application number: PCT/EP2017/063609
(87) International publication number: WO 2017/215972

(56) References cited:
- US-A1- 2010 036 369
- US-A1- 2010 145 328
- US-A1- 2010 286 681
- US-A1- 2012 035 688
- US-A1- 2013 072 920

## Description

### FIELD OF THE INVENTION

The invention relates to electrosurgical devices for treating biological tissue with microwave energy. In particular it relates to electrosurgical devices capable of generating sufficient power to ablate biological tissue. The invention may find particular use in procedures that use a surgical scoping device, e.g. endoscope, gastroscope, bronchoscope or the like, but the principle is equally applicable to devices used in laparoscopic and open procedures.

### BACKGROUND TO THE INVENTION

The use of microwave energy in the treatment of biological tissue is well known. However, it remains a challenge to deliver microwave energy in a closely controlled manner, primarily due to the effect of losses between the microwave source and applicator structure in contact with tissue. These effects can be particularly problematic in minimally invasive procedures using surgical scoping devices. In such arrangements, the generator is typically located outside the patient, which means that a high power treatment signal needs to be carried along the length of the instrument cord before it can be utilised at the treatment region. Losses in the instrument cord can lead to undesirable endoluminal heating and consequently a limit on the power available at the treatment region, which in turn leads to longer treatment times.

US 9,023,025 discloses an electrosurgical system in which a microwave amplifier is removably mounted in the handle of an electrosurgical instrument. Further electrosurgical systems are disclosed in documents US2010/145328 A1, US2012/035688 A1 and US2010/286681 A1.

### SUMMARY OF THE INVENTION

The invention is defined in the appended set of claims. At its most general, the present invention contemplates integrating at least the output stage of a microwave signal generating line up into a distal portion of an electrosurgical instrument. In other words, the invention provides an electrosurgical instrument in which the main amplifier for getting microwave power is located in the same region at a radiating structure for delivering that power.

The invention is based fundamentally on the development of wide band-gap semiconductor materials, such as GaN and GaN-based alloys, which offer the ability to fabricate devices that are active at RF and microwave frequencies. In particular, it has be found that AlGaN/GaN power High Electron Mobility Transistors (HEMTs) have significantly improved output power performance that can be utilised for electrosurgery.

GaN HEMTs have a large material band gap - this allows them to be operated at high drain voltages and high power density, i.e. 10W/mm³ or more. This enables smaller devices to be used to achieve a given power density and to be integrated into a range of radiating structures, including radiating spatula shaped structures for ESD/EMR procedures or integration into miniature cameras that can be swallowed or inserted through a natural orifice.

In some embodiments, the entire microwave generating line up can be incorporating into the instrument, which means that only DC power needs to be introduced. The microwave power losses and associated drawbacks present in known devices can therefore be avoided.

According to the invention, there may be provided an electrosurgical instrument for ablating biological tissue, the electrosurgical instrument comprising a tissue treatment portion for contacting the biological tissue to be treated, the tissue treatment portion having: an microwave power source comprising a power amplifier arranged to generate microwave energy suitable for ablating biological tissue; and a radiating structure connected to the microwave power source and configured to deliver the microwave energy into the biological tissue. The device for generating the microwave power for treatment is therefore located with the radiating structure, which can minimise the distance that the high power signal needs to be transported before it is delivered into biological tissue.

The tissue treatment portion may be one part of a larger instrument. For example, the instrument may include a handle for holding and/or controlling operation of the instrument. The handle may be connected to the tissue treatment portion by one or more cables, e.g. to transfer control signals or to transmit mechanical forces for steering or tuning the instrument as discussed below. In some case, these cables may be long, e.g. when the instrument is to be used with a surgical scoping device. By providing the power amplifier in the tissue treatment portion, the invention ensures that it is not necessary to transport a high power microwave signal between the handle and the radiating structure.

The power amplifier may include a wide band-gap semiconductor transistor, such as a GaN-based HEMT. To ensure efficient transfer of power between an input signal and the output microwave energy, the power amplifier may have a class F design.

The microwave power source in the tissue treatment portion may comprise generator circuitry for driving the power amplifier. Although in principle this circuitry could be located elsewhere, e.g. in the handle, it is desirable to provide it in the tissue treatment portion to maximise power conversion efficiency.

The generator circuitry may comprise: an oscillator arranged to receive a DC input signal and generate a microwave frequency signal; and a driver amplifier arranged to receive the microwave frequency signal and generate an input signal for the power amplifier. The oscillator may be a voltage controlled oscillator (VCO) or a dielectric resonant oscillator (DRO). The driver amplifier may be any suitable MMIC device. The generator circuitry may include a signal modulator, e.g. switch, for pulsing the output for the oscillator. In some example, it may be desirable to drive the power amplifier with pulses of microwave energy. The modulator may be provided to control these pulses. The generator circuitry may include a signal attenuator, e.g. before the driver amplifier, to enable the magnitude of the input signal for the power amplifier to be controlled.

The tissue treatment portion may include a power source, e.g. for providing the DC input signal. It need not be essential for the power source to be part of the tissue treatment portion. It may for example be located in the handle discussed above. However, by including the power source in the tissue treatment portion, the invention may be embodied as a completely stand alone device that can be fully inserted or otherwise introduced into a patient's body, i.e. without the need to maintain physical connections to a external portion. For example, the invention may be embodied as an endoscopy capsule or the like.

The power source may be a cell for providing DC power. For example, the power source may comprise a battery (e.g. a lithium ion battery), supercapacitor or fuel cell. Alternatively or additionally, the power source may comprise a coupling unit for inductively or magnetically coupling power from an external supply.

In order to further improve the efficiency of energy delivery into tissue, the power amplifier may be connected to the radiating structure in the tissue treatment portion by a tunable transmission line. The transmission line may have any suitable structure; it may be a coaxial line, or a microstrip-based structure. The tunability of the structure may be provided by enabling the transmission line to exhibit an adjustable electrical length.

In one embodiment, the tunable transmission line may comprise an axially extendable coaxial structure, e.g. having two separate mating coaxial section that can move axially relative to each other. The coaxial section may include axially expandable dielectric material and extendable conductive portions to maintain the transmission line functionality has its length is adjusted.

In another embodiment, the power amplifier may be movable relative to the radiating structure along the tunable transmission line to adjust the electrical length thereof.

The tissue treatment portion may be enclosed by a sleeve, e.g. to protect the microwave power source components. The sleeve may be retractable to expose the radiating structure.

The radiating structure may comprise any suitable antenna for launching microwave power into biological tissue. The radiating structure may be bipolar, i.e. comprise a first conductor and second conductor separated by a dielectric material in a manner that causes them to act as an antenna for the microwave energy. The radiating structure may be a coaxial structure or a microstrip based structure. For example, the radiating structure may comprise a dipole antenna, a slotted antenna, or the like.

The instrument of the invention may be specifically adapted for use with a surgical scoping device. For example, the instrument may comprise a handle connected to the tissue treatment portion via a flexible shaft, wherein the tissue treatment portion forms a distal end assembly suitable for insertion (e.g. dimensioned to fit) through an instrument channel of a surgical scoping device.

The instrument of the invention may find application in a wide range of procedures. For example, it may be used in a tissue resection device suitable for using the gastrointestinal tract. The radiating structure may be an omni-directional antenna suitable for tumour ablation. As discussed above, the instrument may be part of a capsule or PILL camera for vision and ablation in the GI tract. The invention may find use in a implantable device for insertion into bone (or elsewhere), especially the embodiments that can be energized from an external source. These implanted devices may be used to control the growth of tumours or other conditions where a mass of tissue grows at a higher rate than normal. One particular application here is to control the growth of brain tumours.

In this specification "microwave" may be used broadly to indicate a frequency range of 400 MHz to 100 GHz, but preferably the range 1 GHz to 60 GHz. Specific frequencies that have been considered are: 915 MHz, 2.45 GHz, 3.3 GHz, 5.8 GHz, 10 GHz, 14.5 GHz and 24 GHz.

Similarly references to a "conductor" or "conductive" material herein are to be interpreted as meaning electrically conductive unless the context makes clear that another meaning is intended.

### BRIEF DESCRIPTION OF THE DRAWINGS

Embodiments of the invention are discussed below with reference to the accompanying drawings, in which:
Fig. 1 is a schematic diagram showing an electrosurgery system for use with an embodiment of the invention;
Fig. 2 is a schematic diagram showing a distal end assembly having an integrated microwave source that is an embodiment of the invention;
Fig. 3 is a schematic diagram of components in the distal end assembly of Fig. 2;
Figs. 4A and 4B are schematic side views illustrating operation of a tunable transmission line that can be used in embodiments of the invention;
Figs. 5A and 5B are schematic cross-sectional views illustrating operation of a tunable coaxial transmission line that can be used in embodiments of the invention;
Fig. 6 is a schematic diagram showing components in an example output stage that can be used in embodiments of the invention; and
Fig. 7 is a schematic cross-sectional view through a radiating structure that can be used in embodiments of the invention.

### DETAILED DESCRIPTION; FURTHER OPTIONS AND PREFERENCES

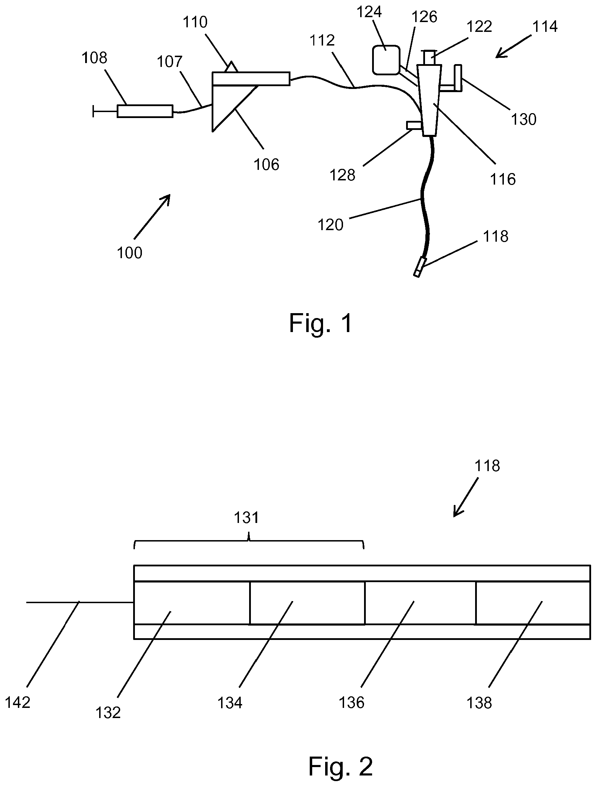

Fig. 1 is a schematic diagram of an electrosurgery system 100 in which the present invention may be used.

The system comprises a surgical scoping device 114, such as an endoscope, gastroscope, laparoscope or the like. The surgical scoping device 114 comprises a body 116 having a number of input ports and an output port from which an instrument cord 120 extends. The instrument cord 120 comprises an outer jacket which surrounds a plurality of lumens. The plurality of lumens convey various things from the body 116 to a distal end of the instrument cord 120. One of the plurality of lumens is an instrument (working) channel. A flexible shaft 112 is insertable along the entire length of the instrument (working) channel. Other lumens may include a channel for conveying optical radiation, e.g. to provide illumination at the distal end or to gather images from the distal end. The body 116 may include a eye piece 122 for viewing the distal end. In order to provide illumination at the distal end, a light source 124 (e.g. LED or the like) may be connected to the body 116 by an illumination input port 126.

At a proximal end of the flexible shaft 112 there is a handle 106, which may be connected to receive a fluid supply 107 from a fluid delivery device 108, such as a syringe, although this need not be essential. If needed, the handle 106 can house an instrument control mechanism that is operable by sliding a trigger 110, e.g. to control longitudinal (back and forth) movement of one or more control wires or push rods (not shown). If there is a plurality of control wires, there may be multiple sliding triggers on the handle to provide full control.

At a distal end of the flexible shaft 112, there is a distal assembly 118 (not drawn to scale in Fig. 1) that is shaped to pass through the instrument channel of the surgical scoping device 114 and protrude (e.g. inside the patient) at the distal end of the instrument cord 120. The distal end assembly includes an active tip for delivering microwave energy into biological tissue, as discussed in more detail below.

The structure of the distal assembly 118 may be arranged to have a maximum outer diameter equal to or less than 2.0 mm, e.g. less than 1.9 mm (and more preferably less than 1.5 mm) and the length of the flexible shaft can be equal to or greater than 1.2 m.

The body 116 may includes a DC power source 128 that is connected to delivery DC energy to the distal end assembly 118 along the flexible shaft, e.g. using suitable leads. The DC power source may be a battery (e.g. a lithium ion battery), supercapacitor or a fuel cell mounted in the body 116. In another example, the DC power source 128 may be a coupling unit arranged to inductively or magnetically couple energy into the device from a remote source (not shown). In this case, the coupling unit may comprise internal rectification and filtering to obtain a DC signal from coupled energy.

In yet further examples, the DC power source may be part of the distal end assembly, in which case leads extending along the instrument channel are not required.

It may be desirable to control the position of at least the distal end of the instrument cord 120. The body 116 may include a control actuator 130 that is mechanically coupled to the distal end of the instrument cord 120 by one or more control wires (not shown), which extend through the instrument cord 120. The control wires may travel within the instrument channel or within their own dedicated channels. The control actuator 130 may be a lever or rotatable knob, or any other known catheter manipulation device. The manipulation of the instrument cord 120 may be software-assisted, e.g. using a virtual three-dimensional map assembled from computer tomography (CT) images.

Fig. 2 shows a distal end assembly 118 that incorporates an electrosurgical instrument that is an embodiment of the invention. The electrosurgical instrument of the invention is characterised by having components that generate microwave energy in situ, so it is not necessary to convey a microwave signal along the flexible shaft. The distal end assembly 118 therefore comprises a microwave generator line up 131 having generator circuitry 132 and an output stage 134 that comprises a GaN-based transistor, e.g. a high-density GaN HEMT device. The microwave generator line up 131 is connected to a radiating structure 138 via a transmission line 136. As discussed below, the transmission line 136 may be adjustable to improve efficiency of power delivery from the microwave generator line up to the radiating structure 138.

The distal end assembly 118 may be encased in a jacket or sleeve 140. This may protect the components of the distal end assembly as they are inserted along the instrument channel. The sleeve 140 may be an over-mould of polymer formed directly onto the device or a deposited insulating coating.

In Fig. 2 the sleeve 140 is shown as having an open end. An open-ended sleeve may be used where it is desirable to have the radiating structure 138 in direct contact with the tissue. On open-ended sleeve may also be useful if the distal end assembly is also configured to deliver radiofrequency (RF) energy, where it is desirable to have direct tissue contact.

However, having an open sleeve need not be essential. For example, the jacket may have closed distal end. It may be completely sealed to form a barrier between the tissue and internal components. The distal end may be shaped, e.g. to assist in positioning the device or shaping the output field.

At a proximal side of the generator circuitry 132 there may be other interface circuitry and/or cables, e.g. a cable 142 for conveying DC power to the generator circuitry from the DC power source 128. As discussed above, the DC power source 128 may be in the body 116 of the scoping device, or it may be part of the distal end assembly 118.

The whole circuitry for the microwave generator line up 131 may be mounted on a flexible substrate. This may make the distal end assembly 118 flexible. As a result, it could be bent, flexed or deformed into pre-defined shapes (i.e. flat, cylindrical) as desired.

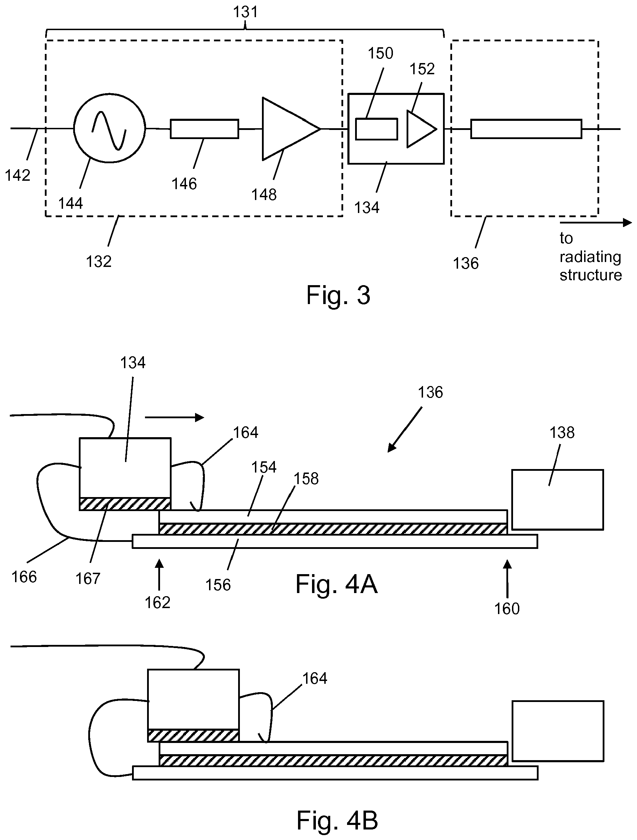

Fig. 3 is a schematic view showing further components of the microwave generator line up 131. The generator circuitry 132 comprising an oscillator 144 for outputting a microwave signal, e.g. having a frequency of 1 GHz or more, preferably 5.8 GHz or more. The oscillator 144 may be a voltage controlled oscillator (VCO) or a dielectric resonator oscillator (DRO). The oscillator 144 may receive the DC power discussed above as an input. The output from the oscillator 144 may be pulsed by a modulator 146. The output from the oscillator 144 is provided to a driver amplifier 148, which is arranged to generate an input signal for the output stage 134. The driver amplifier 148 may be any suitable MMIC device. The line up may include an attenuator (not shown) to provide control over the amplitude of the signal delivered to the output stage 134. The output stage 134 itself may comprises a biasing circuit 150 and a GaN-based transistor 152 configured as a power amplifier. The output stage may include circuitry (not shown) to protect the output stage components from signal reflects back from the radiating structure. For example, a circulator may be mounted on a forward path from the GaN-based transistor. The circulator may divert reflected power to a dump load. However, this protection structure is not essential because GaN-based structure can be robust enough to cope.

Fig. 3 illustrates an embodiment in which the oscillator 144 and modulation switch 146 are part of the distal end assembly 118. This need not be necessary (although it is desirable). For example, the oscillator 144 and modulation switch 146 may be located in or at the body 116 of the surgical scoping device. In another example, the whole generator circuitry 132 (i.e. including the driver amplifier 148) may be located at a proximal distance from the distal end assembly, e.g. in the body 116. Thus, the input signal for the output stage 134 may be transmitted along the instrument channel.

To illustrate, one example may comprise a DRO with an output power of 10dBm (1 mW) and a MMIC with a gain of 20dB located in the body of the scoping device. Even if the insertion loss of the cable is 10dB in this scenario, there would still be 20dBm (100 mW) available at the distal end assembly. In this example, the output stage may comprise a second MMIC followed by the GaN-based transistor 152. If the second MMIC has a gain of 10dB and a high density GaN device a gain of 10dB, then there will be 40dBm (10 W) available for delivery.

The transmission line 136 may be any suitable structure for conveying the microwave power generated by the output stage 134 to the radiating structure. For example, both coaxial (including waveguide) structures and microstrip structures may be used, as explained in more detail below.

The transmission line 136 may provide an impedance matching function to improve the efficiency of energy delivery to the radiating structure. For example, on board or in situ matching may be achieved by varying the distance (e.g. in fractions or wavelength or phase) between the load (which will most likely include the radiating structure) and the output stage, e.g. output junction of the high density GaN device. In this example, there may also be provided a variable parallel connected or shunt stub arranged to null out the susceptance once the length of line has moved the load onto the unity conductance circle.

In another example, the transmission line may be an inline or series connected quarter wavelength transformer. This structure matches the real impedance of the load to the real part of the complex impedance of the output of the GaN transistor.

Examples of coaxial-based structures (including non-coaxial waveguides, such as square waveguides) that can be used for the transmission line, may be as follows:
(a) a short length structure, e.g. having a length equal to or less than one tenth of a wavelength. This would result in minimal loss and reduced impedance mismatch (if transmission line and output stage impedance are non-identical) compared to a longer arbitrary length of line.
(b) a quarter wavelength (or odd multiple of a quarter wavelength) transmission line may be used. The geometry of the transmission line may be selected such that the impedance of the transmission line acts as an impedance transformer between the output stage and the radiator. This would result in improved matching to non-ideal loads.
(c) a half wavelength (or odd multiple of a half wavelength) transmission line may be used. This results in the impedance observed by the output stage (at the interface between the output stage and transmission line) being substantially equivalent to the input impedance of the radiating structure. As a result, the impedance (and the geometry) of the transmission line is less constrained.

The same principles of the above coaxial-based transmission lines may also apply to transmission lines based on microstrip, stripline, or coplanar line structures.

Adapting the transmission line so that it can help with matching can be achieved by enabling relative axial movement between components at opposite ends of the transmission line, thereby to alter its effective length.

In one example, this can be done by permitting axial movement of one or more internal components within the sleeve 140 with respect to the radiating structure 138. Such movement can be enabled by sliding or flexible joints (interference fit or soldered flexible conductors) within the distal transmission line 136, i.e. between output stage 134 and radiating structure 138.

Figs. 4A and 4B show one example of an adjustable transmission line. In this example, the transmission line 136 is a microstrip structure having an upper conductor 154 and a lower conductor 156 formed on a layer of dielectric material 158. The radiating structure 138 is shown schematically connected to a distal end 160 of the transmission line 136. This connection may be fixed.

The output stage 134 is mounted at a proximal end 162 of the transmission line 136. Flexible leads 164, 166 are provided to connect the relevant parts of the output stage to the upper conductor 154 and lower conductor 156 respectively.

The output stage 134 is adapted to slide relative to the radiating structure 138 along the transmission line 136. An insulating layer 167 may be provided on output stage 134 to prevent electrical connection with the transmission line 136 for occurring at unwanted locations. Suitable mechanisms for controlling movement are discussed below.

As shown in Fig. 4B, as the output stage 134 slides along the transmission line 136, the position at which the lead 164 connects to the first conductor 154 also changes. This movement allows for changes in phase due to effective changes in transmission line length to the radiating structure 138. As a result, the impedance can be shifted to be purely real i.e. no reactance (capacitive or inductive components). Improved impedance matching allows for more efficient coupling of energy from the generator circuitry to the radiating structure, and therefore also more efficient energy delivery into the tissue surrounding the radiating structure.

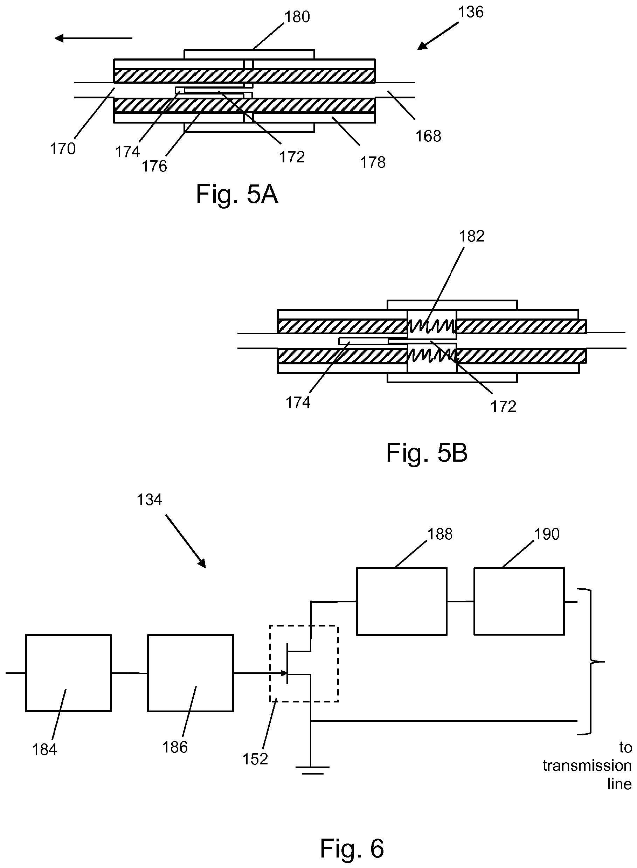

Figs. 5A and 5B show cross-sectional views through a sliding coaxial transformer junction that can be used to alter the length of a coaxial transmission line. In this example, the transmission line 136 comprises a coaxial structure having an inner conductor formed from a distal part 168 and a proximal part 170. These parts have mutual engagement elements that slide over each other in an axial direction to enable the length of the inner conductor to vary without breaking the conductive path. In the specific example shown, the distal part has a male element 172 that mates with a female element 174 in the proximal part 170.

An axially expandable dielectric material 176 (e.g. formed from a spring, foam or the like) separates the inner conductor from a coaxial outer conductor 178. The outer conductor 178 is in two parts that correspond to and move with the distal part 168 and proximal part 170 of the inner conductor. A conductive sheath 180 lies over the junction between the parts of the outer conductor to ensure that the conductive path is maintained. The outer conductor 170 and sheath 180 of the coaxial transformer may be flexible or rigid. In an alternative embodiment, the outer conductor 170 may comprise a coiled or braided spring, such that as the gap 182 opens, the spring extends to maintain the conductive path. This structure can facilitate impedance matching, e.g. by allowing greater changes to geometry and spacing between inner and outer conductors.

As shown in Fig. 5B, as the transmission line is axially extended, e.g. by pulling the proximal part 170 in the proximal direction, the dielectric material 176 expands into the gap 182 to maintain the integrity of the transmission line structure as the distal and proximal parts are drawn apart.

The structure shown in Figs. 5A and 5B thus forms a collapsible quarter wave transformer.

In other examples. the transmission line may include both a microstrip structure and a coaxial based structure.

Control of the axial movement may be performed mechanically, e.g. by the operator sliding an internal or external push/pull wire or sleeve using a slider 110 on the handle 106. Alternatively, the axial movement may be actuated electrically for example with an electromechanical switch. Precise movement may be achieved through the use of a micro stepper motor or similar, which can be mounted in the handle 106 in the body 116 of the scoping device.

Fig. 6 is a schematic diagram of the components in an output stage 134 that can be used in an embodiment of the invention. As discussed above, the output stage 134 uses an high density GaN-based HEMT 152 as an amplifier for an input signal received from the generator circuitry 132. Whilst any suitable amplifier configuration may be used, it may be desirable to bias the output transistor 152 using schemes other than the traditional class A, B, A-B or C in order to optimise the DC to microwave power conversion efficiency.

In particular, if the DC power source is also located in the distal end assembly, e.g. as a stand alone battery or as a coupling unit for drawing energy from an external source, it may be desirable to configure the device to take the power added efficiency (PAE) close to its theoretical limit. Fig. 6 shows an output stage 134 in which this is achieved by setting up a class F biasing structure. This structure may be able to achieve up to 90% PAE. In one example, a open stub with a physical length set to 1/12 of the wavelength of the frequency is mounted at the output of the transistor. This arrangement may increases the efficiency of operation by affecting the drain voltage and drain current waveforms. In an ideal scenario, a zero output voltage is wanted when the drain voltage is maximum or a zero current is wanted when the drain current is maximum. This ensure that the power dissipated in the GaN device is related to the current flowing from the drain to the source and the voltage across the drain-source junction.

The class F structure in Fig. 6 provides a first resonant circuit (e.g. a LC or tank circuit) 184 at an input to the GaN-based HEMT 152 and a second resonant circuit 190 at an output thereof. Each resonant circuit 184, 190 has a respective matching circuit 186, 188 (e.g. a series LC circuit). The device is biases near or at cut-off, in a similar manner to class B operation.

In order to increase the efficiency in terms of the amount of microwave power produced at the output to DC and input microwave signal at the input, it is desirable to operate the GaN device using a scheme other than the standard linear Class A scheme, i.e. Class B, AB, C, D, E or F.

The efficiency of an amplifier is limited by the characteristics of the transistors used in the design. If class F design is used then it is theoretically possible to achieve 100% efficiency, but this assumes that the transistor is an ideal current source. In practice, it should be possible to achieve up to 10% power added efficiency (PAE) using a class F arrangement.

The second resonant circuit 190 is configured to shape the output waveform based on the load appearing as a short circuit to even harmonics (i.e. short circuit at 2f₀, where f₀ is the resonant frequency of the circuit) and as an open circuit to odd harmonics (i.e. open circuit at 3f₀). Accordingly, the drain voltage waveform is shaped towards a square wave whereas the drain current is shaped such that it resembles a half-wave sinusoidal waveform.

The first resonant circuit 184 assists in ensuring that the device is driven by square wave pulses.

The radiating element 138 shown schematically in Fig. 2 may take any form suitable for delivering microwave energy. The radiating element 138 may be bipolar, i.e. include a first conductor and a second conductor arrangement to act as an antenna for radiating microwave energy into biological tissue. Structures of this type that are suitable for insertion down the instrument channel of a surgical scoping device include spatula type probes such as those described in WO 2011/010089, WO 2012/095653 and WO 2014/006369. Alternatively, dipole or slotted antennas can be used. Dielectric radiators, e.g. similar to those disclosed in WO 2008/044013 may also be used.

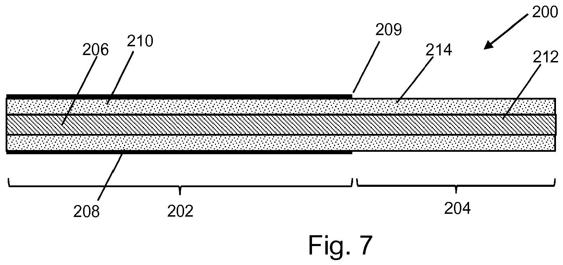

Fig. 7 is a cross-sectional view of the distal end of an radiating structure 200 that can used in the distal assembly 118 to deliver microwave energy into biological tissue. The radiating structure 200 comprises a coaxial cable 202 that is connected at its proximal end to the transmission line structure discussed above to receive microwave energy from the output stage. The coaxial cable 202 comprises an inner conductor 206, which is separated from an outer conductor 208 by a first dielectric material 210. The coaxial cable 202 is preferably low loss for microwave energy. A choke (not shown) may be provided on the coaxial cable to inhibit back propagation of microwave energy reflected from the distal end and therefore limit backward heating along the device.

The coaxial cable 202 terminates at its distal end with a radiating tip section 204. In this embodiment, the radiating tip section 204 comprises a distal conductive section 212 of the inner conductor 206 that extends before a distal end 209 of the outer conductor 208. The distal conductive section 212 is surrounded at its distal end by a dielectric tip 214 formed from a second dielectric material, which can be the same or different from the first dielectric material 210. The length of the dielectric tip 214 is shorter than the length of the distal conductive section 212.

The coaxial cable 202 and radiating tip section 204 may have a biocompatible outer sheath (not shown) formed over their outermost surfaces. The outer sheath 218 may be formed from a biocompatible material.

The dielectric tip 214 may have any suitable distal shape, e.g. any of dome shape, cylindrical, conical, etc. A smooth dome shape may be preferred because it increases the mobility of the antenna as it is manoeuvred through small channels.

Although the radiating structure 200 shown in Fig. 7 is based on a coaxial structure, this need not be essential. For example, the radiating structure may be flat and formed on microstrip type structure. Alternatively, the radiating structure may be formed on an expandable substructure, e.g. an inflatable balloon or forceps-type hinged structure such as that disclosed in WO 2015/097472.

The microwave energy delivered by the radiating structure may have a power level suitable for tissue ablation. This may be the primary use of the electrosurgical invention of the invention. However, the instrument may also operate at lower power levels, e.g. to measure properties of the tissue at the distal end of the device.

In one example, the device may be arranged to operate at a higher frequency for measurement than for ablation. For example, ablation may be performed at 5.8 GHz, whilst measurement may be performed at 60 GHz or 77 GHz. These higher frequencies can enable sensitive measurements to be obtained. By integrating the microwave source into the distal end assembly, measurement uncertainty caused by phase and magnitude variation due to cable bending and flexure is avoided.

The higher frequency measurement signal may be obtained by coupling off a low power signal from the oscillator 144 and provided a separate local oscillator having a frequency selected to enable the signals to be mixed up to a frequency for measurement. This arrangement may enable ablation and measurement to take place simultaneously.

## Claims

1. An electrosurgical instrument for ablating biological tissue, the electrosurgical instrument comprising:
a tissue treatment portion for contacting the biological tissue to be treated, and
a handle connected to the tissue treatment portion via a flexible cable, wherein the tissue treatment portion forms a distal end assembly suitable for insertion through an instrument channel of a surgical scoping device;
wherein the tissue treatment portion comprises:
a microwave power source comprising a power amplifier arranged to generate microwave energy suitable for ablating biological tissue; and
a radiating structure connected to the microwave power source and configured to deliver the microwave energy into the biological tissue, wherein
the power amplifier is connected to the radiating structure by a tunable transmission line.

2. An electrosurgical instrument according to claim 1, wherein the power amplifier includes a wide band-gap semiconductor transistor.

3. An electrosurgical instrument according to claim 1 or 2, wherein the power amplifier includes a GaN-based HEMT.

4. An electrosurgical instrument according to any preceding claim, wherein the power amplifier has a class F design.

5. An electrosurgical instrument according to any preceding claim, wherein the microwave power source comprises generator circuitry for driving the power amplifier.

6. An electrosurgical instrument according to claim 5, wherein the generator circuitry comprises:
an oscillator arranged to receive a DC input signal and generate a microwave frequency signal; and
a driver amplifier arranged to receive the microwave frequency signal and generate an input signal for the power amplifier.

7. An electrosurgical instrument according to any preceding claim, wherein the tissue treatment portion includes a power source.

8. An electrosurgical instrument according to claim 7, wherein the power source is a cell for generating DC power.

9. An electrosurgical instrument according to claim 7, wherein the power source comprises a coupling unit for inductively or magnetically coupling power from an external supply.

10. An electrosurgical instrument according to claim 1, wherein the tunable transmission line has an adjustable electrical length.

11. An electrosurgical instrument according to claim 1 or 10, wherein the tunable transmission line comprise an axially extendable coaxial structure.

12. An electrosurgical instrument according to claim 1 or 10, wherein the power amplifier is movable relative to the radiating structure along the tunable transmission line to adjust the electrical length thereof.

13. An electrosurgical instrument according to any preceding claim, wherein the tissue treatment portion is enclosed by a sleeve.

## Patentansprüche

1. Elektrochirurgisches Instrument zur Ablation von biologischem Gewebe, wobei das elektrochirurgische Instrument Folgendes umfasst:
einen Gewebebehandlungsabschnitt zum Berühren des zu behandelnden biologischen Gewebes und
einen Griff, der über ein flexibles Kabel mit dem Gewebebehandlungsabschnitt verbunden ist, wobei der Gewebebehandlungsabschnitt eine distale Endanordnung bildet, die zum Einführen durch einen Instrumentenkanal einer chirurgischen Untersuchungsvorrichtung geeignet ist;
wobei der Gewebebehandlungsabschnitt Folgendes umfasst:
eine Mikrowellenleistungsquelle, umfassend einen Leistungsverstärker, der angeordnet ist, um Mikrowellenenergie zu erzeugen, die zur Ablation von biologischem Gewebe geeignet ist; und
eine Strahlungsstruktur, die mit der Mikrowellenleistungsquelle verbunden ist und ausgelegt ist, um die Mikrowellenenergie in das biologische Gewebe abzugeben, wobei
der Leistungsverstärker durch eine abstimmbare Übertragungsleitung mit der Strahlungsstruktur verbunden ist.

2. Elektrochirurgisches Instrument nach Anspruch 1, wobei der Leistungsverstärker einen Halbleitertransistor mit breitem Bandabstand umfasst.

3. Elektrochirurgisches Instrument nach Anspruch 1 oder 2, wobei der Leistungsverstärker einen GaN-basierten HEMT umfasst.

4. Elektrochirurgisches Instrument nach einem der vorangegangenen Ansprüche, wobei der Leistungsverstärker ein Design der Klasse F aufweist.

5. Elektrochirurgisches Instrument nach einem der vorangegangenen Ansprüche, wobei die Mikrowellenleistungsquelle eine Generatorschaltung zum Ansteuern des Leistungsverstärkers umfasst.

6. Elektrochirurgisches Instrument nach Anspruch 5, wobei die Generatorschaltung Folgendes umfasst:
einen Oszillator, der angeordnet ist, um ein GS-Eingangssignal zu empfangen und ein Mikrowellenfrequenzsignal zu erzeugen; und
einen Treiberverstärker, der angeordnet ist, um das Mikrowellenfrequenzsignal zu empfangen und ein Eingangssignal für den Leistungsverstärker zu erzeugen.

7. Elektrochirurgisches Instrument nach einem der vorangegangenen Ansprüche, wobei der Gewebebehandlungsabschnitt eine Leistungsquelle umfasst.

8. Elektrochirurgisches Instrument nach Anspruch 7, wobei die Leistungsquelle eine Zelle zum Erzeugen von GS-Leistung ist.

9. Elektrochirurgisches Instrument nach Anspruch 7, wobei die Leistungsquelle eine Kopplungseinheit zum induktiven oder magnetischen Koppeln von Leistung aus einer externen Versorgung umfasst.

10. Elektrochirurgisches Instrument nach Anspruch 1, wobei die abstimmbare Übertragungsleitung eine einstellbare elektrische Länge aufweist.

11. Elektrochirurgisches Instrument nach Anspruch 1 oder 10, wobei die abstimmbare Übertragungsleitung eine axial verlängerbare Koaxialstruktur umfassen kann.

12. Elektrochirurgisches Instrument nach Anspruch 1 oder 10, wobei der Leistungsverstärker relativ zur Strahlungsstruktur entlang der abstimmbaren Übertragungsleitung bewegbar ist, um deren elektrische Länge einzustellen.

13. Elektrochirurgisches Instrument nach einem der vorangegangenen Ansprüche, wobei der Gewebebehandlungsabschnitt von einer Hülse umschlossen ist.

## Revendications

1. Instrument électro-chirurgical destiné à ablater du tissu biologique, l'instrument électro-chirurgical comprenant :
une partie de traitement de tissu destinée à être mise en contact avec le tissu biologique à traiter, et
une poignée raccordée à la partie de traitement de tissu via un câble flexible, dans lequel la partie de traitement de tissu forme un ensemble d'extrémité distale destiné à être inséré à travers un canal d'instrument d'un dispositif d'examen chirurgical ;
dans lequel la partie de traitement de tissu comprend :
une source de puissance micro-ondes comprenant un amplificateur de puissance conçu pour générer une énergie micro-ondes destinée à ablater du tissu biologique ; et
une structure rayonnante raccordée à la source de puissance micro-ondes et configurée pour délivrer l'énergie micro-ondes dans le tissu biologique, dans lequel
l'amplificateur de puissance est raccordé à la structure rayonnante par une ligne de transmission accordable.

2. Instrument électro-chirurgical selon la revendication 1, dans lequel l'amplificateur de puissance inclut un transistor à semi-conducteurs à large bande interdite.

3. Instrument électro-chirurgical selon la revendication 1 ou 2, dans lequel l'amplificateur de puissance inclut un HEMT à base de GaN.

4. Instrument électro-chirurgical selon l'une quelconque des revendications précédentes, dans lequel l'amplificateur de puissance a une conception de classe F.

5. Instrument électro-chirurgical selon l'une quelconque des revendications précédentes, dans lequel la source de puissance micro-ondes comprend un ensemble de circuits générateurs destiné à exciter l'amplificateur de puissance.

6. Instrument électro-chirurgical selon la revendication 5, dans lequel l'ensemble de circuits générateurs comprend :
un oscillateur conçu pour recevoir un signal d'entrée de courant continu et générer un signal d'hyperfréquence ; et
un amplificateur d'excitation conçu pour recevoir le signal d'hyperfréquence et générer un signal d'entrée pour l'amplificateur de puissance.

7. Instrument électro-chirurgical selon l'une quelconque des revendications précédentes, dans lequel la partie de traitement de tissu inclut une source de puissance.

8. Instrument électro-chirurgical selon la revendication 7, dans lequel la source de puissance est une pile destinée à générer une puissance continue.

9. Instrument électro-chirurgical selon la revendication 7, dans lequel la source de puissance comprend une unité de couplage destinée à coupler de manière inductive ou magnétique une puissance provenant d'une alimentation externe.

10. Instrument électro-chirurgical selon la revendication 1, dans lequel la ligne de transmission accordable a une longueur électrique réglable.

11. Instrument électro-chirurgical selon la revendication 1 ou 10, dans lequel la ligne de transmission accordable comprend une structure coaxiale extensible axialement.

12. Instrument électro-chirurgical selon la revendication 1 ou 10, dans lequel l'amplificateur de puissance est mobile par rapport à la structure rayonnante le long de la ligne de transmission accordable pour régler la longueur électrique de celle-ci.

13. Instrument électro-chirurgical selon l'une quelconque des revendications précédentes, dans lequel la partie de traitement de tissu est entourée par une gaine.
